# EUROPÄISCHE PATENTANMELDUNG

(11) **EP 0 788 783 A1**
(43) Veröffentlichungstag der Anmeldung: **13.08.1997**
(21) Anmeldenummer: 97101579.7
(22) Anmeldetag: 01.02.1997
(51) Int. Cl.: A61F 5/41

(54) **Modifizierter Cockring**

(30) Priorität: 08.02.1996 DE 19604465
(71) Anmelder: CRIVELLARO, Jürgen, D-22145 Stapelfeld (DE)
(72) Erfinder: Crivellaro, Jürgen, 22145 Stapelfeld (DE)
(74) Vertreter: Siewers, Gescha, Dr.

(57) **Zusammenfassung**

Die Erfindung betrifft eine Vorrichtung zur Steigerung und Verlängerung der Erektion. Der erfindungsgemäße modifizierte Cockring ist dadurch gekennzeichnet, daß er einen eiförmig ausgebildeten Umfang besitzt, wobei in einer besonders vorteilhaften Ausführungsform, diejenige Hälfte, in der der Abstand des Umfangs zur Längsachse geringer ist, in spiegelbildlicher Weise Einbuchtungen aufweist. Die Erfindung betrifft des weiteren einen Cockring, der dadurch gekennzeichnet ist, daß er eine Verschlußöffnung aufweist, die vorteilhafterweise im oberen Peniswurzelbereich angebracht ist. Weiterhin betrifft die Erfindung einen Cockring, der dadurch gekennzeichnet ist, daß seine Innenseite konvex gekrümmt ist, wobei es sich herausgestellt hat, daß es besonders vorteilhaft ist, wenn die gekrümmte Innenseite zusätzlich mittig umlaufend einen Sattel aufweist.

## Beschreibung

Die Erfindung betrifft einen modifizierten Cockring.

Cockringe, oder auch Pressringe, sind in vielen Kulturen seit Jahrhunderten in Verwendung und werden häufig eingesetzt, um die Potenzschwäche des Mannes zu beheben. Sie umschließen gleichzeitig Glied und Hodensack und sind daher von reinen Penisringen zu unterscheiden. Cockringe können aus elastischem Material, wie beispielsweise Gummi, oder aus nicht elastischem Material, wie beispielsweise Stahl, oder, wie häufig im japanischen Kulturkreis anzutreffen, aus Porzellan oder Elfenbein bestehen. Der durch den Cockring ausgeübte Druck verhindert einerseits den Abfluß venösen Blutes, ist aber andererseits nicht so groß, daß der starke Zufluß arteriellen Blutes davon beeinträchtigt wird. Cockringe unterstützen zudem die Bindegewebshülle von Glied und Hodensack und fördern insbesondere durch den im Bereich der Gliedwurzel ausgeübten Druck die Entstehung und den Erhalt einer Erektion.

Herkömmliche Cockringe sind zumeist konzentrisch ausgebildet und in unterschiedlichen Durchmessergrößen erhältlich. Der Innenquerschnitt ist häufig kreisrund geformt, kann aber auch annähernd rechteckig sein. Die Materialbreite wird im allgemeinen so bemessen, daß Stauchungen im Hodenbereich vermieden werden, und ist daher selten größer als etwa 3 cm.

Potenzschwierigkeiten beim Mann sind nach Erkenntnissen der Sexualmediziner weit verbreitet und scheinen ständig, insbesondere im höheren Alter zuzunehmen und führen sehr oft zu psychischen und sozialen Problemen. Ein Grund für die Zunahme scheint beispielsweise auch in der verbreiteten Gabe von Betablockern gegen Herz- und Blutdruckbeschwerden bei Männern im Alter von etwa ab 40 zu liegen. Nach neuesten Erkenntnissen leiden mehr als die Hälfte aller Männer über 40 unter zeitweiliger oder allgemeiner Impotenz. Die Verwendung von Cockringen wird daher auch von Sexualmedizinern bei Potenzschwäche empfohlen.

Konzentrische Cockringe haben den Nachteil, daß eine ideale Anpassung an die jeweiligen anatomischen Gegebenheiten nur in den seltensten Fällen optimal gelingt. Um die gewünschte, die Erektion fördernde Wirkung zu erzielen, ist ein gewisser Mindestdruck auf den Wurzelbereich des Penisschaftes notwendig. Deshalb darf der Durchmesser des Cockrings auf keinen Fall zu groß dimensioniert sein, da ansonsten der Rückfluß des venösen Blutes nicht mehr gestaut wird. Eine optimale Druckwirkung im Penisschaftbereich ist aber häufig mit den herkömmlichen konzentrischen Cockringen nur auf Kosten einer sehr hohen Presswirkung im Hodenbereich zu erzielen. Da bei sexueller Erregung in der Regel auch das Hodensackvolumen zunimmt, wird dieser Bereich bei herkömmlichen Cockringen einem erheblichen Druck ausgesetzt, der mitunter als schmerzhaft, zumindest aber als irritierend empfunden wird, und daher der gewünschten Wirkung abträglich ist. Aus den gleichen Gründen wird das Anlegen eines derartigen Cockrings als unangenehm empfunden und kann sogar zuweilen schmerzhafte Hautquetschungen zur Folge haben.

Es besteht daher noch ein Bedarf an Cockringen, die geeignet sind, den erforderlichen Druck im Bereich der Gliedwurzel aufzubauen, ohne jedoch gleichzeitig im Hodenbereich einen zu starken Druck zu erzeugen, die sich unkompliziert und, ohne Schmerzen zu verursachen, anlegen lassen und die beim Anlegen und Gebrauch Quetschungen vermeiden helfen.

Aufgabe der Erfindung ist es deshalb, einen Cockring zu entwickeln, der die genannten Nachteile nicht mehr aufweist und in idealer Weise geeignet ist, eine schwache Erektion zu steigern bzw. die Dauer einer Erektion zu verlängern.

Erfindungsgemäß wird deshalb ein einteiliger Cockring vorgeschlagen, dessen Umfang eiförmig ausgebildet ist. Hierdurch wird eine nahezu optimale Anpassung an die anatomischen Gegebenheiten erzielt. Besonders vorteilhaft ist dabei eine Konstruktion, die in demjenigen Abschnitt des Cockrings, in dem dessen Umfang den geringeren Abstand zur Längsachse aufweist, in spiegelbildlicher Weise Einbuchtungen besitzt. Ein derartiger erfindungsgemäßer Cockring ist im oberen Teil dem Durchmesser eines erigierten Gliedes angepaßt, während das untere, größere Ringsegment eine optimalere Anpassung an den volumenreicheren Hodenbereich gestattet. Auf diese Weise wird ein angemessen hoher Druck auf die Penisschaftwurzel erzeugt, der ausreicht, den venösen Blutabfluß zu verhindern, ohne jedoch gleichzeitig im Hodenbereich unangenehme Druckempfindungen zu verursachen, wobei der in diesem Bereich erzeugte Druck allerdings immer noch ausreicht, um als stimulierend empfunden zu werden. Der erfindungsgemäße Cockring ist bevorzugt in einem Stück gearbeitet. In einer möglichen Ausführungsform ist der Umfang des erfindungsgemäßen Cockrings an keiner Stelle durchtrennt.

Erfindungsgemäß wird des weiteren ein Cockring vorgeschlagen, der dadurch gekennzeichnet ist, daß er eine Verschlußöffnung besitzt. Die einander gegenüberliegenden Endungen sind in der Weise als Verschlußsegmente ausgeformt, daß sie innenseitig einen nahtlosen Abschluß erlauben. Der erfindungsgemäße Cockring mit Verschluß hat den Vorteil, daß er, ohne Schmerzen zu verursachen, völlig unproblematisch angelegt werden kann, da ein Überstülpen entfällt. In einer besonderen Ausführungsform wird die Verschlußöffnung in dem Ringbereich angebracht, in dem die Längsachse des erfindungsgemäßen Cockrings dasjenige Segment schneidet, das den geringeren Krümmungsradius besitzt. Alternativ kann der Verschluß aber auch in einem Bereich angebracht werden, in dem die Längsachse das Segment mit dem größeren Krümmungsradius schneidet. Die Verschlußsegmente sind vorzugsweise in gegengleicher Form als ineinandergreifende Haken ausgeformt und befinden sich in geöffnetem Ruhezustand in einem bestimmten Abstand voneinander, wodurch ein einfaches Verschließen durch Zusammendrücken der Längsseiten des erfindungsgemäßen Cockrings gelingt. Im geschlossenen Zustand zeigt der Cockring insbesondere auf der Innenseite keine Nuten oder Überstände und unterscheidet sich demgemäß in seiner Form auch nicht von einem einstückig gearbeiteten Cockring, der keine Verschlußöffnung besitzt. Die Verschlußhaken greifen ineinander und werden durch die dem Cockringmaterial eigene Steifigkeit gegeneinander gedrückt, so daß auch bei starker Beanspruchung nicht damit gerechnet werden muß, daß der Verschluß sich löst oder verschiebt. Hautquetschungen werden so ausgeschlossen.

Erfindungsgemäß wird des weiteren ein Cockring vorgeschlagen, der dadurch gekennzeichnet ist, daß seine Innenseite konvex ausgebildet ist. In einer bevorzugten Ausführungsform verfügt die Innenseite des erfindungsgemäßen Cockrings, die selber bereits leicht konvex gewölbt sein kann, über einen mittig verlaufenden Sattel. Gegenüber herkömmlichen runden oder aber vollständig abgeflachten Innenseiten ist bei dem erfindungsgemäßen Cockring von Vorteil, daß der Druck auf den Wurzelbereich des Gliedes durch die besondere Formgebung der Innenseite graduierlich aufgebaut wird. Die erfindungsgemäße Ausführungsform wird generell als angenehmer und auch stimulierender empfunden, was u.a. darauf zurückzuführen ist, daß der Rückfluß venösen Blutes auf besonders effektive Weise unterbunden wird und daß Hautquetschungen vermieden werden.

Die erfindungsgemäßen Cockringe bestehen aus physiologisch unbedenklichen Kunststoffen. Bevorzugt wird ein Material mit einer Härte verwendet, das der Umfangsform eine angemessene Steifigkeit verleiht, so daß diese auch bei äußerer Krafteinwirkung erhalten bleibt. Die verwendeten Kunststoffe sollten eine Shorehärte im Bereich zwischen etwa 45A bis 80A und 30D bis 90D aufweisen. Die erfindungsgemäßen Cockringe können auch aus Keramik, Elfenbein, Stahl oder Gummi bestehen.

Die Breite der erfindungsgemäßen Cockringe beträgt 0,5 bis 3 cm, vorzugsweise jedoch etwa 1 cm.

Die Erfindung wird im folgenden anhand von Zeichnungen näher erläutert:

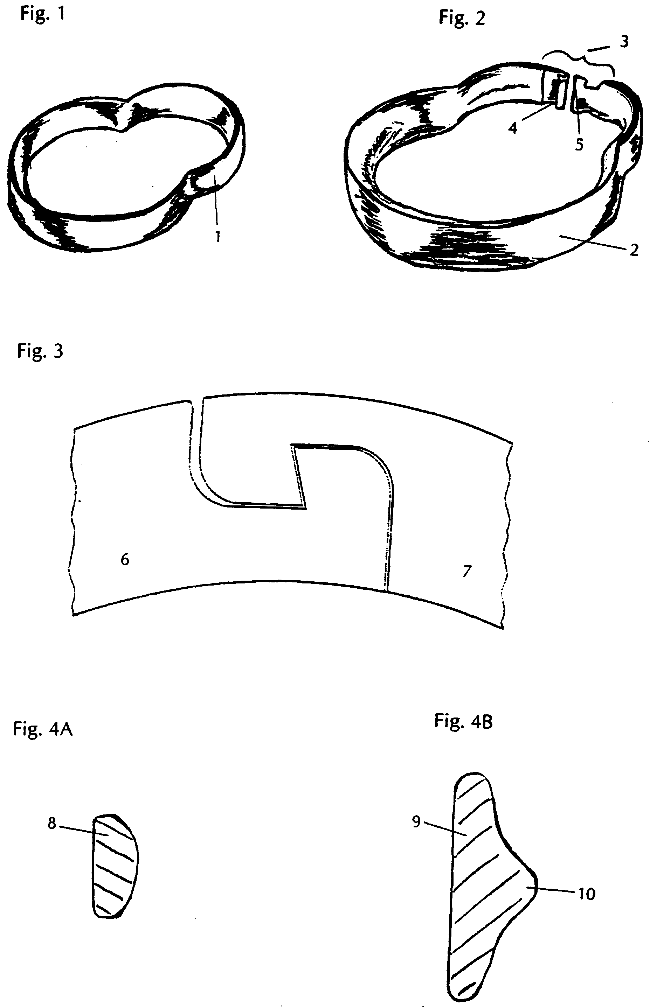

Figur 1 zeigt einen erfindungsgemäßen Cockring (1) in Draufsicht.
Figur 2 zeigt einen erfindungsgemäßen Cockring (2) mit einer im Penisschaftbereich angebrachten Verschlußöffnung (3). Der erfindungsgemäße Cockring ist so gearbeitet, daß seine Verschlußsegmente (4, 5) sich im geöffneten Ruhezustand nicht berühren. Aufgrund der dem verwendeten Kunststoffmaterial eigenen Steifigkeit werden die ineinandergreifenden Verschlußhaken gegeneinander gedrückt, so daß eine besonders sichere Arretierung gelingt.
Figur 3 zeigt skizzenhaft die Verschlußsegmente A (6) und B (7) in Draufsicht in geschlossenem Zustand. Die Verschlußsegmente sind paßgenau gearbeitet, so daß gewährleistet ist, daß die Oberflächenform der Innenseite erhalten bleibt.
Figur 4A zeigt einen Querschnitt (8) durch einen erfindungsgemäßen Cockring, bei dem die Innenseite in konvexer Weise ausgeformt ist.
Figur 4B zeigt einen Querschnitt (9), bei dem die leicht gekrümmte Innenseite zusätzlich mittig eine sattelförmige Ausbuchtung (10) aufweist.

## Patentansprüche

1. Cockring, dadurch gekennzeichnet, daß er einen eiförmig ausgebildeten Umfang besitzt.

2. Cockring nach Anspruch 1, dadurch gekennzeichnet, daß er in der Hälfte, in der der Abstand des Umfangs zur Längsachse geringer ist, in spiegelbildlicher Weise Einbuchtungen aufweist.

3. Cockring nach Anspruch 1 oder 2, dadurch gekennzeichnet, daß er in einem Stück gearbeitet ist.

4. Cockring nach Anspruch 1 bis 3, dadurch gekennzeichnet, daß der Umfang des erfindungsgemäßen Cockrings an keiner Stelle durchtrennt ist.

5. Cockring nach Anspruch 1 bis 3, dadurch gekennzeichnet, daß er eine Verschlußöffnung besitzt.

6. Cockring nach Anspruch 5, dadurch gekennzeichnet, daß die Verschlußöffnung in etwa in dem Bereich angebracht ist, in dem seine Längsachse dasjenige Ringsegment schneidet, das den geringeren Krümmungsradius besitzt.

7. Cockring nach Anspruch 5, dadurch gekennzeichnet, daß die Verschlußöffnung in etwa in dem Bereich angebracht ist, in dem seine Längsachse dasjenige Ringsegment schneidet, das den größeren Krümmungsradius besitzt.

8. Cockring nach Anspruch 1 bis 7, dadurch gekennzeichnet, daß seine Innenseite konvex gekrümmt ist.

9. Cockring nach Anspruch 8, dadurch gekennzeichnet, daß die gekrümmte Innenseite mittig einen Sattel besitzt.

10. Cockring nach Anspruch 1 bis 9, dadurch gekennzeichnet, daß er aus physiologisch unbedenklichen Kunststoffen besteht.

11. Cockring nach Anspruch 10, dadurch gekennzeichnet, daß der Kunststoff eine Shorehärte zwischen etwa 45A - 80A und 30D - 90D besitzt.
